# EUROPEAN PATENT SPECIFICATION

(11) **EP 3 939 564 B1**
(45) Date of publication and mention of the grant of the patent: **09.10.2024**
(21) Application number: 20770312.5
(22) Date of filing: 09.03.2020
(51) Int. Cl.: A61K 9/00, A61K 47/32, A61K 47/38, A61K 47/10, A61K 9/70, A61K 31/192, A61P 29/00

(54) **CATAPLASMA PATCH FOR RELIEVING TOPICAL PAIN**
KATAPLASMAPFLASTER ZUR LINDERUNG VON TOPISCHEN SCHMERZEN
TIMBRE DE CATAPLASME POUR SOULAGER UNE DOULEUR TOPIQUE

(30) Priority: 12.03.2019 KR 20190028209
(43) Date of publication of application: 19.01.2022
(73) Proprietor: Wooshin Labottach Co., Ltd., Seoul 08390 (KR)
(72) Inventor: NAM, Taek Soo, Seoul 07341 (KR)
(74) Representative: Louis Pöhlau Lohrentz
(86) International application number: PCT/KR2020/003285
(87) International publication number: WO 2020/184946

(56) References cited:
- CN-A- 105 960 234
- KR-B1- 100 351 088
- KR-B1- 100 648 445
- KR-B1- 100 844 103
- KR-B1- 101 478 566
- KR-B1- 101 717 699
- KR-B1- 101 772 140
- KR-B1- 101 772 140
- KR-B1- 102 022 336
- US-B2- 9 861 598

## Description

### Technical Field

The present invention relates to a system for delivering a drug into the skin using the principle of iontophoresis. Specifically, the present invention relates to kit comprising a cataplasma for local pain relief and effectively delivering a pain relief drug of the cataplasma into the skin using an iontophoretic device and an electrically conductive cream without skin pain or redness.

### Background Art

### [Local Pain Relief]

Drugs that are used for pain relief include ibuprofen, loxoprofen, ketoprofen, fenoprofen, dexketoprofen, dexibuprofen, flurbiprofen, pranoprofen, and the like.

The present invention relates to a kit comprising a cataplasma composition for local pain relief comprising a profen drug for pain relief. Specifically, the present invention relates to relieving pain by applying a pain-relieving cataplasma to a specific area in need of pain relief and then allowing a drug of the cataplasma to penetrate into the skin. Profens commonly have a carboxyl group (COOH) functional group, and thus are classified as drugs that can be negatively charged when ionized in an aqueous solution.

### [Cataplasma Preparation]

Profen drugs for pain relief have a very low solubility in water. Thus, conventional products are formulations, such as gel cream or dry patches, which are made based on oil or other hydrophobic solvents rather than based on water.

In the present invention, a profen drug for pain relief that has low water solubility is applied to a water-based cataplasma. Accordingly, a solubilizer is used to dissolve the profen drug for pain relief in water.

### [Iontophoresis]

A transdermal administration method may have limitations in effectiveness due to poor skin penetrability of the drug used. To overcome these limitations, methods based on electric propulsion are used to induce effective skin penetration of an ionic, weakly ionic or nonionic drug when the drug is transdermally administered. For example, iontophoresis, electroosmosis, electroporation, etc. are methods of allowing ions of a drug molecule or a solvent molecule containing a drug to move with electric current.

Among the methods based on electric propulsion, iontophoresis is the most representative method. This is a method of more quickly delivering a drug into the skin by giving the same charge to the ionized molecule in the flow of electric current between positive and negative electrodes.
KR 100 648 445 B1 is directed to a cataplasm composition of the loxoprofen containing and manufacturing method thereof. US 9,861,598 B1 discloses a ketoprofen lysine salt-containing aqueous patch.
CN 105 960 234 A is directed to a ketoprofen-containing poultice.
KR 101 772 140 B1 discloses an iontophoresis device for drug delivery and method for manufacturing the same.

### DISCLOSURE

### Technical Problem

First, the present invention is intended to develop a kit comprising a cataplasma formulation based on water, which has excellent adhesive strength, enables a large amount of a drug to penetrate for a long time, and does not affect the patient's activity after the cataplasma patch is attached to the skin.

After a gel cream formulation is applied to the skin, it adheres to clothes or others articles on the skin, and thus interferes with long-term penetration of the drug and limits the patient's activity. A dry patch has a very low adhesive strength because the surface thereof consists of an oil component. In particular, the dry patch has a disadvantage that the adhesive strength thereof completely disappears when sweat comes out of the patient's pores or water occurs. This disadvantage also limits the patient's activity.

Nevertheless, in a conventional art, profen drugs were inevitably formulated as dry patches because these drugs are hydrophobic. In addition, no attempt has been made to formulate these drugs as aqueous cataplasma patches.

However, for obtaining a kit of the present invention, a cataplasma formulation is prepared, which is based on water and comprises a hydrophilic polymer containing a carboxyl group. The hydrophilic polymer containing a carboxyl group combines with water on the skin and creates adhesion. According to this principle, an adhesive cataplasma is prepared.

Second, the present invention is intended to apply a hydrophobic drug to a cataplasma formulation and optimize the type and content of the composition forming the cataplasma formation in order to ensure effective skin penetration of the drug.

In addition, in order for a hydrophobic or lipid-soluble drug to be dissolved in water, a solubilizer is necessary. A surfactant may also be used, but the surfactant is likely to form bubbles during a preparation process, and the bubbles may remain after preparation and reduce the contact area with the skin. In addition, the surfactant is basically a good component that dissolves dead skin cells, but may cause irritation to the skin. Since the cataplasma formulation used in the kit of the present invention is basically intended to adhere to the skin for at least 4 hours, it should be avoided that the patient's skin is exposed to the surfactant for a long time.

In addition, the carboxyl group-containing hydrophilic polymer in the cataplasma is also used to crosslink a polymer. The content of the crosslinked polymer, the density of crosslinking, etc. affect the skin penetration of the drug, and hence it is necessary to optimize the content of the hydrophilic polymer, the content of the crosslinking agent, and an agent for controlling the crosslinking rate.

Third, the present invention is intended to effectively enhance introduction of the drug in the cataplasma by an iontophoresis technique, without being limited to the cataplasma composition.

Cataplasma based on water has a nearly neutral pH or a weakly acidic pH. When a hydrophobic drug is exposed to such a pH condition, only a small amount of the drug is ionized and dissolved. However, when electrical energy is to be applied through an iontophoretic device, several effects happen.

First, a small amount of the ionized drug permeates into the skin. In addition, depending on the pH and ionic strength conditions in the cataplasma, the drug that was not originally ionized is secondarily ionized to maintain the chemical equilibrium state. This ionized drug additionally permeates into the skin. In this way, ionization continues to proceed, and as a result, an effect of allowing the drug to continuously penetrate the skin happens.

Then, as electrical energy is to be applied to the skin, an electronic channel is formed in the epidermis of the skin. There are dead skin cells on the epidermis of the skin and an outer shell under the dead skin cells. The outer shell is composed of a phospholipid structure between skin cells.

In addition, phospholipids are arranged to form a bilayer between cells. Since this phospholipid structure has a strong hydrophobic property, it is difficult for a hydrophilic substance to pass through the phospholipid structure. However, when electrical energy is to be applied, the alignment of the bilayer is changed to an unaligned form, so that the hydrophilic substance can easily pass through the bilayer.

Fourth, the present invention is intended to apply the iontophoresis technique to the cataplasma only for a short time.

When the iontophoresis technique is to be applied for a long time, it may cause irritation such as redness on the skin. In addition, when the ability of the cataplasma to supply water and ions decreases with the passage of time, the overall resistance increases, which may cause heat generation and seriously burns.

### Technical Solution

To solve the above problem, the present invention provides a kit according to claim 1 comprising inter alia a cataplasma composition and an appropriate iontophoresis technique.

Specifically, the technical solution of the present invention is as follows:

A Kit according to claim 1 comprising a cataplasma composition comprising: a profen drug; a hydrophilic polymer; and a solubilizer, wherein the hydrophilic polymer is at least one selected from the group consisting of partially neutralized polyacrylate, and sodium carboxymethylcellulose.

"Partially neutralized" as used herein means that the H ions of COOH of the polyacrylate are neutralized by substitution with Na ions or the like. Preferably, a 50% or 70% neutralized polyacrylate may be used.

According to the invention the profen drug is a drug having a specific structural formula of profen, and is ibuprofen, loxoprofen, ketoprofen, dexibuprofen, flurbiprofen, or pranoprofen.

According to the invention the solubilizer is at least one of propylene glycol, polyethylene glycol, L-menthol, Pharmasolve and N-methyl-2-pyrrolidone.

The cataplasma composition, preferably, further comprising a crosslinking agent, wherein the crosslinking agent is aluminum glycinate.

The cataplasma composition, preferably, further comprising a crosslinking co-agent, wherein the crosslinking co-agent is tartaric acid.

The cataplasma composition, preferably, further comprising an excipient, wherein the excipient is at least one of glycerin, titanium dioxide, polysorbate, kaolin, methylparaben, propylparaben, sodium metabisulfite, and uric acid.

7. The Kit of the present invention uses a cataplasma patch in which the cataplasma composition is applied to a nonwoven fabric.

8. The cataplasma patch used in the kit of the present invention has a pH of 5 to 6.

Preferably, the iontophoretic device is usable with one hand by wetting the cataplasma composition with water, placing the negative electrode of the iontophoretic device in contact on the wet composition, and bringing the positive electrode of the iontophoretic device into contact with the hand.

Preferably, the kit comprising: the cataplasma composition; an electrically conductive gel composition comprising an ionic polymer; and an iontophoretic device, wherein the ionic moiety of the ionic polymer in the electrically conductive gel composition is selected from among an anionic moiety or a cationic moiety, the anionic moiety is at least one selected from the group consisting of carboxylate, nitrate, phosphate, and sulfate, the cationic moiety is at least one selected from the group consisting of amine salt, imine salt, and ammonium salt, the polymer is at least one selected from the group consisting of carboxylate, nitrate, phosphate, and sulfate, and the iontophoretic device is usable with one hand by bringing the negative electrode of the iontophoretic device into contact with the electrically conductive gel composition applied onto the cataplasma composition and bringing the positive electrode of the iontophoretic device into contact with the hand.

Preferably, the ionic polymer is at least one selected from among an acrylamide/sodium acryloyldimethyl taurate copolymer, carbomer, alginate, carrageenan, chitosan, and polyethyleneimine.

### Advantageous Effects

One embodiment of the kit of the present invention provides a cataplasma composition for pain relief, which has improved drug administration efficiency compared to a conventional one, and to be used with an appropriate iontophoresis method.

In addition, according to one embodiment of the present invention, more effective pain relief compared to conventional one may be provided to a patient.

### Brief Description of Drawings

FIG. 1 shows a circuit diagram of an iontophoretic device according to one embodiment of the kit of the present invention.
FIG. 2 shows a circuit diagram of the human body skin and internal resistance.
FIG. 3 shows the transdermal permeation amount of ibuprofen contained in a cataplasma patch.
FIG. 4 shows the transdermal permeation amount of ibuprofen contained in a cataplasma patch and ibuprofen contained in a commercially available formulation.
FIG. 5 shows the transdermal permeation amount of loxoprofen contained in a cataplasma patch.
FIG. 6 compares the transdermal permeation amount of ibuprofen between water was applied and when an electrically conductive gel was applied.

### Mode for Invention

The present invention relates to a kit using a cataplasma composition, which has excellent adhesive strength and enables a pain relief drug to effectively penetrate into the skin. The present invention includes the use of an iontophoretic device for more effective drug penetration.

### [Cataplasma Patch for Local Pain Relief]

The patch used in the kit of the present invention adopts a cataplasma composition. The term "cataplasma" has the same technical meaning as described in a conventional dictionary, and refers to a composition such a pain relief patch. Components and combinations thereof, which show the features of the cataplasma, are known, and this "cataplasma formulation" may contain any additive components within the range that does not impair the features of cataplasma disclosed in the general rules of the Korean Pharmacopoeia.

The cataplasma may be composed of a composition comprising a hydrophilic polymer, a solubilizer, a polyhydric alcohol, and a crosslinking agent, as well as other functional additives as needed. In addition, the cataplasma composition of the present invention may further comprise an excipient, a curing agent, a crosslinking buffer, a crosslinking co-agent, an antioxidant, a preservative, and a pigment.

However, the composition used in the kit of the present invention is characterized by comprising at least one selected from the group consisting of partially neutralized polyacrylate, and sodium carboxymethylcellulose, among hydrophilic polymers that can be generally contained in cataplasma compositions. "Partially neutralized" used regarding the hydrophilic polymer means that the H ions of COOH of the polyacrylate are neutralized by substitution with Na ions or the like. Preferably, 50% or 70% neutralized polyacrylate may be used. The above-described hydrophilic polymer is preferred for the implementation of a cataplasma formulation containing a pain relief drug, and is also preferred in terms of the flow of electric current.

The content of the above-described hydrophilic polymer is preferably 3 wt% to 7 wt% based on the total weight of the cataplasma in terms of water capture and crosslinking effects.

The composition ratio of components in the cataplasma patch used in the kit according to the present invention was compared based on the permeation amount of the profen drug through a transdermal permeation experiment performed using a diffusion cell in hairless mice, and the effect thereof was evaluated while comparing the physical states of other patches.

It is also important that the cataplasma simply contains the drug in terms of the physical state of the formulation. If crosslinking is not done properly, the drug and other components come out to the outside rather than a target skin area, and thus it is not possible to achieve uniform delivery of the drug from the cataplasma.

Specifically, to control physical properties, the proportions of the crosslinking agent, the hydrophilic polymer, the crosslinking buffer and the crosslinking co-agent are adjusted, and the curing agent and the excipient, which are additives, correct physical properties. A substance such as an excipient not only imparts physical stability, but also is used as a chemical stabilizer because it functions as a drug carrier.

The polyhydric alcohol that is used in the present invention may be, but not limited to, glycerin. The content of the polyhydric alcohol is preferably 20 wt% to 40 wt% based on the total weight of the cataplasma in terms of water retention and formulation formation effects.

The crosslinking agent that is used in the present invention may be, but not limited to, aluminum glycinate. The content of the crosslinking agent is preferably 0.01 wt% to 0.3 wt% in terms of crosslinking effects.

In the present invention, the solubilizer may be at least one selected from among propylene glycol, polyethylene glycol, L-menthol, Pharmasolve and N-methyl-2-pyrrolidone. The content of the solubilizer is preferably 1 to 20 wt% based on the total weight of the cataplasma in terms of the skin penetration effect of the drug.

In the present invention, the crosslinking buffer may be at least one selected from among EDTA-Na. The content of the crosslinking buffer is preferably 0 to 1 wt% based on the total weight of the cataplasma in terms of the effect of delaying a crosslinking reaction in the cataplasma.

In the present invention, the crosslinking co-agent may be at least one selected from among tartaric acid. The content of the crosslinking co-agent is preferably 0 to 2 wt% based on the total weight of the cataplasma in terms of accelerating a crosslinking reaction in the cataplasma.

In the present invention, the moisturizing agent may be at least one selected from among glycerin. The content of the moisturizing agent is preferably 20 to 40 wt% based on the total weight of the cataplasma in terms of the moisture retention effect of the cataplasma formulation.

In the present invention, the thickener may be at least one selected from among kaolin. The content of the thickener is preferably 0.1 to 5 wt% based on the total weight of the cataplasma in terms of the effect of ensuring the physical rigidity of the cataplasma formulation.

In the present invention, the excipient may be at least one selected from among titanium dioxide, polysorbate, methylparaben, propylparaben, sodium metabisulfite, and uric acid. The content of the excipient is preferably 0.01 to 1 wt% based on the total weight of the cataplasma in terms of the appearance and chemical stability effect of the cataplasma formulation.

Additives such as an antioxidant, a preservative and a pigment function to preserve the cataplasma after cataplasma preparation rather than during cataplasma preparation, and may be selected from among general components. An antioxidant is added to prevent discoloration and decomposition, and a preservative is added to prevent decomposition. Titanium dioxide acts as a UV blocker, and is also used as a white pigment.

The drug that is used in the present invention is a profen drug. The profen drug is a drug having a specific structural formula, and any profen drug may be used without limitation, as long as it can be polarized when ionized in an aqueous solution so that iontophoresis may be used. In the kit of the present invention ibuprofen, loxoprofen, ketoprofen, dexibuprofen, flurbiprofen or pranoprofen is used.

The content of the profen drug may vary slightly depending on the type of drug used, but may be 0.1 to 20 wt% based on the total weight of the cataplasma in terms of the anesthetic effect thereof.

The patch used in the kit of the present invention is prepared by applying the above-described cataplasma composition to a nonwoven fabric.

The nonwoven fabric preferably has absorption and adhesive properties in terms of a uniform application effect. The above-described cataplasma composition is preferably coated on the nonwoven fabric to a thickness of 1 mm to 2 mm in terms of uniform application.

A known preparation method may be used to prepare the patch. For example, the patch may be prepared by coating a nonwoven fabric with the cataplasma composition through an aging process at a temperature of 40 to 50°C under moisture conditions in a constant-temperature and constant-humidity chamber device.

The patch used in the kit according to the present invention is characterized in that it may be effectively attached to a local area requiring anesthesia, because it has excellent adhesive strength even if it does not contain a particular adhesive component.

In addition, the present invention is to be used by attaching the patch to a local area, and then applying an electrically conductive gel or spraying water onto the outer surface of the nonwoven fabric and allowing an electric current to flow using an iontophoretic device placed thereon. That is, the present invention is characterized in that, even though the electric current is generated on the nonwoven fabric, transmission of the electric current is considerably smooth. Perhaps it is assumed that the composition used in the kit according to the present invention and the content ratio of the components thereof play an auxiliary role in effectively transmitting the electric current, even though the exact cause is unknown.

When water is sprayed onto the outer surface of the nonwoven fabric, water may be used in an amount of 5 to 10 g/cm².

### [Iontophoretic Device]

For the use of the iontophoretic device, an electric circuit in any form is to be formed in the cataplasma patch and the skin so that an electric current flows. The cataplasma patch used in the kit of the present invention is characterized in that it is applied to a non-woven fabric. Since the non-woven fabric is a non-electrically conductive material, an electric current cannot flow through the nonwoven fabric unless the nonwoven fabric is wetted with a liquid containing an electrolyte. According to Ohm's law, the inability of electric current to flow means that resistance is high. If resistance is high, heat is generated in the resistor, causing skin irritation.

To sufficiently wet the nonwoven fabric of the cataplasma, water containing an electrolyte may also be used, but as a preferred example, an electrically conductive gel is used. The electrically conductive gel is a gel containing an ionic hydrophilic polymer. The ionic hydrophilic polymer may be one of an acrylamide/sodium acryloyldimethyl taurate copolymer, carbomer, alginate, carrageenan, chitosan, and polyethyleneimine.

The iontophoretic device used in the kit of the present invention generates an electric current according to a principle similar to that of a conventional iontophoretic device, but is characterized in that the negative electrode thereof may be brought into contact with the electrically conductive cream applied onto the nonwoven fabric on the cataplasma patch used in the kit according to the present invention and the positive electrode is disposed on the handle of the same device. That is, since the pain relief drug used in the kit of the present invention is negatively charged, the negative electrode is applied to the cataplasma patch, and the positive electrode is used as a ground part.

Under the assumption that the nonwoven fabric of the cataplasma patch used in the kit of the present invention is sufficiently wetted, the voltage is preferably 1 V to 15 V, and the electric current is preferably 0.1 mA/cm² to 1.5 mA/cm². Before presenting the application time, since the intensity of the electric current varies, power (Watt) per unit area is calculated instead of the electric current. The power is a value obtained by multiplying all of the voltage, current, and time. A power value of 1 Watt means a power applied at a voltage of 1 V and an electric current of 1 A for 1 second. The power per unit area may be preferably in the range of 0.01 Watt/cm² to 10 Watt/cm².

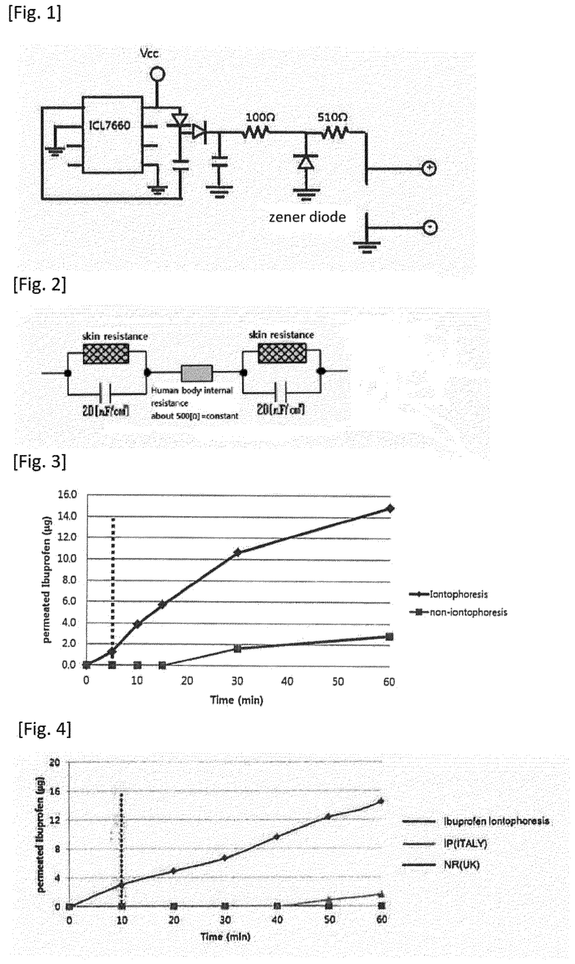

As shown in FIG. 1, a circuit diagram for a local skin anesthesia experiment according to an embodiment of the present invention may comprise one ICL7660 chip, one 10 V Zener diode, two 50 V 4001 diodes, one 100 ohm resistor, one 510 ohm resistor, and two 10 µF capacitors. As a power supply, a 9 V alkaline battery may be used. The ICL7660 chip serves to double the voltage of the power supply. The Zener diode functions to maintain the output voltage of the electrode part at 10 V or 15 V.

The positive electrode of the iontophoretic device of the present invention may be composed of a circular substrate having an area of 4.5 cm², and may be made of at least one material selected from the group consisting of 92.5 to 99% pure stainless steel, platinum, and titanium. These components are preferred because they have low resistance and are not oxidized. More preferably, a titanium substrate coated with platinum may be used.

The positive electrode may have a thickness of 2 mm and may be coated with platinum to a thickness of 0.3 um.

The iontophoretic device used in the kit of the present invention is characterized in that the negative electrode is disposed on the handle. The coated surface of the negative electrode may be composed of at least one selected from the group consisting of silver, copper, silicon and aluminum. These components are preferred because they have low resistance and are not oxidized. More preferably, a coating agent consisting of a mixture of silicon, silver particles and copper particles is used.

A 95% ethanol solution is mixed at a ratio of 1:1 with a coating agent consisting of a mixture of silicon, silver particles and copper particles, and the resulting mixture may be sprayed on the coating surface of the negative electrode. The sprayed mixture is dried slightly at room temperature, and then dried in an oven at 60°C for 30 minutes. At this time, the electrical conductivity of the dried mixture may be similar to that of 92.5% pure silver.

A resistance circuit diagram of the human skin is configured as shown in FIG. 2 below.

The resistance of the skin is about 3000 ohm, which means a dry state. However, in the kit of the present invention, the cataplasma and the electrically conductive gel are to be applied to the skin and the skin is sufficiently washed before an experiment, and hence it can be said that the resistance of the skin decreases to 500 ohm. In addition, it can be considered that the opposite handle also has low resistance, because the handle is gripped by the palm of the hand rather than normal skin and the palm has more sweat glands than the skin of other areas. Therefore, the resistance in the human body during an anesthesia experiment is estimated to be about 2000 to 2500 ohm.

In addition, when a direct current is to be applied to a resistance-capacitance (RC) circuit, the resistance of the capacitor in the circuit almost disappears over time. Therefore, as the resistance gradually decreases after the start of anesthesia, the efficiency of anesthesia may increase.

The iontophoretic device used in the kit according to the present invention may have a current condition of 0.01 to 1.2 mA/cm². When this current condition is satisfied, an anesthetic effect is obtained.

The voltage condition of the iontophoretic device may be 1 to 15 V in terms of an anesthetic effect. Preferably, the voltage may be 10 V. However, even if the voltage is set to 15 V, the system according to the present invention causes little or no skin irritation.

The time for which the iontophoretic device is to be applied may be adjusted according to the situation where anesthesia is required, and may be selected within the range of about 5 to 60 minutes.

The waveform of a current generated by the iontophoretic device may be a constant direct current or a pulsed direct current. This waveform is effective in that it causes little skin irritation.

### [Electrically Conductive Gel]

The electrically conductive gel may comprise an ionic polymer and a surfactant.

The ionic moiety of the ionic polymer may be selected from an anionic moiety or a cationic moiety depending on the properties of the polymer. At this time, the anionic moiety may be at least one selected from the group consisting of carboxylate, nitrate, phosphate and sulfate, and the cationic moiety may be at least one selected from the group consisting of amine salt, imine salt and ammonium salt.

The ionic polymer may be at least one selected from the group consisting of carboxylate, nitrate, phosphate and sulfate. Preferably, sulfate may be selected. More preferably, an acrylamide/sodium acryloyldimethyl taurate copolymer may be selected. The above-described polymer is preferred in terms of electrical conductivity and stability. The content of the ionic polymer is preferably 0.5 wt% to 5 wt% based on the total weight of the composition in terms of dispersion and cream-forming effects.

The surfactant has excellent dispersion and cream-forming effects. As the surfactant, for example, a nonionic surfactant may be used. Preferably, polysorbate 80 or the like may be used in order to minimize skin irritation. The content of the surfactant is preferably 0.5 wt% to 1 wt% based on the total weight of the composition in terms of dispersion and cream-forming effect.

The electrically conductive gel may be prepared by mixing the ionic polymer, the surfactant, and water.

### [Examples]

A skin redness experiment was conducted as follows.

First, the area to be anesthetized is washed with lukewarm water for about 30 seconds to 1 minute. After washing, the area should not be allowed to dry. Alternatively, wet tissue wetted with saline solution is applied to the area. Thereafter, a cataplasma patch is attached. An appropriate amount of either a polymeric electrically conductive gel or water is applied thereon. While the negative electrode part of an iontophoretic device is put thereon, the positive electrode part is properly wrapped and held by hand. The iontophoretic device is turned on, and anesthesia is performed while the device is properly pressed for 5 minutes. After 5 minutes, the iontophoretic device is turned off, the cataplasma patch is removed, and the condition of the skin to which the cataplasma patch was applied is observed.

A transdermal permeation experiment on a pain relief drug was conducted as follows.

A circuit for a transdermal permeation experiment in a hairless mouse is powered by a 4.5 V battery, and a current of about 1.0 mA is allowed to flow with a resistance of 4.3 kohm. A patch is attached onto the skin of the hairless mouse, and then an appropriate amount of an electrically conductive gel or water is applied thereto. Then, a silver plate is put thereon. 5 mL of PBS solution (pH 6.0) is placed in a diffusion cell, and a silver wire is placed in a side opposite the hairless mouse skin to form a positive electrode.

The experiment is performed by drawing 1 mL of the solution every 5 minutes, 10 minutes or 20 minutes after turning on the battery, and supplementing 1 mL of a fresh PBS solution. For analysis of the solution, measurement is performed at 254 nm using a UV detector through HPLC. The HPLC analysis results are collected, and the amount of profen drug permeated as a function of time is determined.

A test for the adhesive strength of the cataplasma was conducted as follows.

The following test method was performed with reference to the vortex test method of the Korean Pharmacopoeia.

The prepared cataplasma patch is cut into 10 cm in width and 14 cm in length. The cut patch is fixed to the test surface by a double-sided tape so that the nonwoven fabric side faces down. The PET film is removed from the cataplasma. An inclined tripod for the vortex test is placed vertically on the horizontal surface of the patch. A ball is placed on the inclined tripod and allowed to roll down. The distance by the ball has moved on the cataplasma patch is measured.

The measurement of the surface pH of the cataplasma patch was performed as follows.

2 g of cataplasma gel is taken, added to 100 mL of distilled water, and mixed sufficiently. The pH of the water is measured.

### [Experiment on Cataplasma Composition]

### Ibuprofen Cataplasma Formulation

Glycerin and various powder-type components required for cataplasma patch preparation are first mixed together, and tartaric acid is added to and dissolved in distilled water. A pain relief drug is mixed with a solubilizer and added to the glycerin layer. The polymer is crosslinked while the distilled water layer is mixed with the glycerin layer.

Table 1 below relates to tests for the degree of crosslinking and physical properties of a cataplasma formulation containing ibuprofen, which is one of pain relief drugs. As the content of ibuprofen is increased, the content of the hydrophilic polymer should also be increased. The reason is that the structure of ibuprofen contains COOH and the hydrophilic polymer also contains COOH. They complete with each other and have the effect of inhibiting the crosslinking reaction of the cataplasma. In addition, the aluminum glycinate crosslinking agent is structurally separated into trivalent aluminum ions and reacts with three COOH groups. The crosslinking agent serves to connect the three COOH groups of the hydrophilic polymer, and the COOH of ibuprofen may also inhibit the crosslinking reaction. For this reason, it is preferable that the content of the crosslinking agent is also increased depending on the content of ibuprofen. This phenomenon may be applied to all profen drugs having a COOH group.

**[Table 1]**

| | Example 1 | Example 2 | Example 3 | Example 4 | Example 5 |
|---|---|---|---|---|---|
| Content of ibuprofen | 0.1% | 1% | 1.43% | 5% | 10% |
| Content of partially neutralized polyacrylate | 5.5% | 5.5% | 5.5% | 5.5% | 55% |
| Content of sodium carboxymethylcellulose | 2.2% | 2.2% | 2.2% | 2.2% | 2.2% |
| Kaolin | 0.5% | 0.5% | 0.5% | 0.5% | 0.5% |
| Propylene glycol | 17% | 17% | 17% | 17% | 17% |
| Glycerin | 25% | 25% | 25% | 25% | 25% |
| Tartaric acid | 0.5% | 0.5% | 0.5% | 0.5% | 0.5% |
| Aluminum glycinate | 0.07% | 0.07% | 0.07% | 0.07% | 0.07% |
| EDTA-Na | 0.03% | 0.03% | 0.03% | 0.03% | 0.03% |

**[Table 2]**

| | Example 6 | Example 7 | Example 8 | Example 9 | Example 10 |
|---|---|---|---|---|---|
| Content of loxoprofen | 0.1% | 1% | 1.43% | 5% | 10% |
| Content of partially neutralized polyacrylate | 5.5% | 5.5% | 5.5% | 5.5% | 55% |
| Content of sodium carboxymethylcellulose | 2.2% | 2.2% | 2.2% | 2.2% | 2.2% |
| Kaolin | 0.5% | 0.5% | 0.5% | 0.5% | 0.5% |
| Propylene glycol | 17% | 17% | 17% | 17% | 17% |
| Glycerin | 25% | 25% | 25% | 25% | 25% |
| Tartaric acid | 0.5% | 0.5% | 0.5% | 0.5% | 0.5% |
| Aluminum glycinate | 0.07% | 0.07% | 0.07% | 0.07% | 0.07% |
| EDTA-Na | 0.03% | 0.03% | 0.03% | 0.03% | 0.03% |

As example of preferred experiments, Example 3 was selected for use in a subsequent experiment on ibuprofen, and Example 9 was selected for use in a subsequent experiment on loxoprofen.

### Experimental Example 1

### Experiment for Measurement of Chemical and Physical Properties of Cataplasma Formulation

To measure the chemical and physical properties of the cataplasma formulations of Examples 1 to 10 among the above Examples, the pH and adhesive strength of each cataplasma formulation were measured.

**[Table 3]**

| | Example 1 | Example 2 | Example 3 | Example 4 | Example 5 |
|---|---|---|---|---|---|
| pH | 5.5 | 5.5 | 5.5 | 5.5 | 5.5 |
| Adhesive strength (cm) | 5.1 cm | 5 cm | 5.1 cm | 5.3 cm | 5.5 cm |

**[Table 3]**

| | Example 6 | Example 7 | Example 8 | Example 9 | Example 10 |
|---|---|---|---|---|---|
| pH | 5.5 | 5.5 | 5.5 | 5.5 | 5.5 |
| Adhesive strength (cm) | 5 cm | 5.3 cm | 5.1 cm | 5.1 cm | 5 cm |

As can be seen in the Tables above, the cataplasma patch used in the kit according to the present invention had a weakly acidic pH of 5 to 6, which is a pH condition under which the cataplasma patch has no adverse effect when attached to the skin. In addition, the cataplasma patch used in the kit according to the present invention showed an adhesive strength of 7 cm or less, indicating that the cataplasma formulation used in the kit according to the present invention has a good physical adhesive strength.

### Application of Iontophoretic device to Ibuprofen Cataplasma Patch

For application of the iontophoretic device, the above-mentioned voltage, current and application time conditions were adopted and an experiment was performed. When a voltage of 4.5 V, a current of 1 mA/cm² and when an application time of 5 min are calculated as power, 1.35 Watt/cm² is obtained. Alternatively, depending on an experiment, 2.7 Watt/cm² was applied for an application time of 10 minutes.

The above-described conditions were selected as preferred conditions and applied to a subsequent transdermal permeation experiment.

### Results of 5-Minute Application of Ibuprofen Iontophoresis and Results of Experiment to Which Ibuprofen Iontophoresis Was Not Applied

The portion up to the red dotted line in FIG. 3 shows the results of applying iontophoresis for only 5 minutes, and the portion up to 60 minutes after the red dotted line shows the results of performing the experiment without iontophoresis. The red line shows the result of applying the ion introduction for 5 minutes, and the blue line shows the result of the experiment in which the ion introduction was not applied. The red continuous line shows the results of applying iontophoresis for 5 minutes, and the blue line shows the results of the experiment to which iontophoresis was not applied.

This difference is related to the onset time. In the experiment to which iontophoresis was applied, a rapid increase was observed at 5 minutes, and in the experiment to iontophoresis was not applied, an increase was observed only after 15 minutes. This suggests that iontophoresis has the effect of quickly starting the permeation of the drug in the cataplasma. In addition, the rate at which the drug penetrates after 5 minutes can be seen by looking at the slope of the continuous line curve in FIG. 3. It can be confirmed that the curve slope of the experiment to which iontophoresis was applied is greater than the curve slope of the experiment to which iontophoresis was not applied. Dramatically, greater effects can be expected when stronger voltage and current conditions and a longer application time are applied. However, since electrical stimulation of the human skin may be severe, appropriate electrical conditions are required.

### Experiment for Comparison of Performance of 10-Minute Application of Iontophoresis

From the blue line in FIG. 4, it can be seen that, when the cataplasma patch used in the kit according to the present invention was applied for 10 minutes, the amount of ibuprofen permeated into the skin increased considerably. On the other hand, from the green line (IP, Italy) and the red line (NR, UK), it can be seen that, even when iontophoresis was applied for 10 minutes, the amount of the ibuprofen drug permeated was considerably small for 60 minutes.

These results varied depending on whether the cataplasma formulation is a hydrophilic formulation or a hydrophobic formulation. Since the hydrophobic formulation is a formulation that does not conduct electricity due to an oil component, the amount of ibuprofen permeated is inevitably small even when the iontophoresis method is applied.

### Experiment for Observation of Skin Redness

In a skin redness experiment, iontophoresis was applied to the cataplasma patch at a voltage of 10 V, a current of 1 mA and a power of 6 Watt/cm² for an application time of 10 minutes. The experiment was conducted in two ways. That is, the following skin conditions were measured: the skin condition immediately after applying the iontophoresis method for 10 minutes; and the skin condition after applying the iontophoresis method for 10 minutes and then attaching only the cataplasma patch for 50 minutes without applying the iontophoresis method. Five or more subjects participated in this experiment.

**[Table 5]**

| | Redness after 10 minutes | Other irritations | Redness after 60 minutes | Other irritations |
|---|---|---|---|---|
| Subject 1 | Not observed | Not observed | Not observed | Not observed |
| Subject 2 | Not observed | Slightly itchy | Not observed | Not observed |
| Subject 3 | Not observed | Not observed | Not observed | Not observed |
| Subject 4 | Not observed | Slightly itchy | Not observed | Not observed |
| Subject 5 | Not observed | Slightly itchy | Not observed | Not observed |

The current condition of the iontophoresis method to be used according to the present invention is a low current condition that is less irritating to the skin, and the hydrophilic cataplasma and the conductive gel together serve to eliminate skin irritation. In addition, the cataplasma patch used in the kit of the present invention is not attached only for 10 minutes, but attached for a long time. Thus, even for a person with sensitive skin, the iontophoresis method is to be applied only for 10 minutes, and then the cataplasma patch continuously lowers the heat of the skin, and the cataplasma patch having a high water content continuously moisturizes the skin. Thus, the cataplasma patch is less burdensome on the skin.

### Results of Application of Iontophoretic device to Loxoprofen Cataplasma

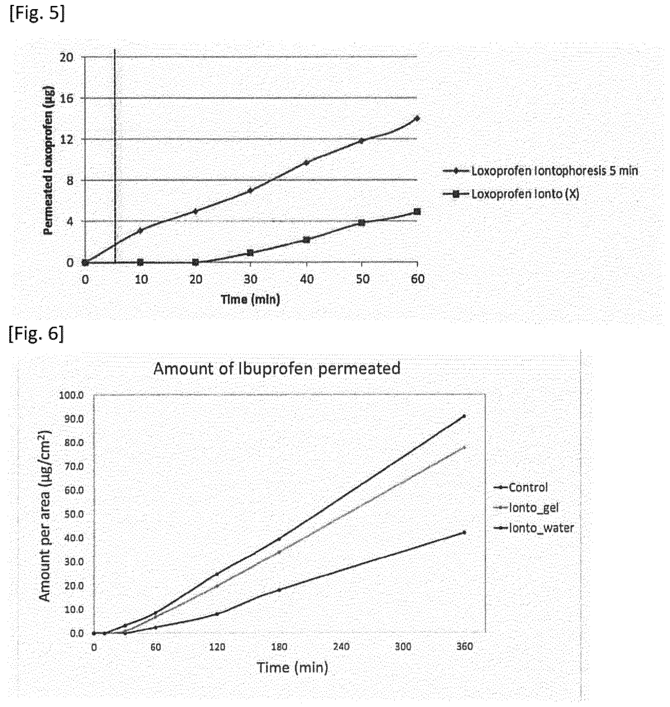

The portion up to the red dotted line in FIG. 5 shows the result of applying iontophoresis only for 5 minutes, and the portion up to 60 minutes after the red dotted line shows the results of the experiment without iontophoresis. The blue curve shows the results of applying iontophoresis for 5 minutes, and the orange curve shows the results of the experiment to which iontophoresis was not applied.

From the results in FIG. 5, it can be seen that the carboxyl group (C00(-)) in the molecule of loxoprofen is negatively charged and the skin permeation of loxoprofen is increased when iontophoresis is applied according to the same principle as ibuprofen.

### Experiment for Comparison of Ibuprofen Permeation Between When Water is Applied and When Electrically Conductive Gel Is Applied

The above-described experiments are experiments conducted using the electrically conductive gel. In this experiment, ibuprofen was used as a representative drug, and the permeation of the drug when water was applied instead of the electrically conductive gel was tested.

The experimental method was the same as the above-described experiment shown in FIG. 4, except that water was applied instead of the electrically conductive gel.

As a result, it was confirmed that the amount of ibuprofen permeated into the skin was further increased when the cataplasma patch was wetted with water than when the electrically conductive gel was applied (see FIG. 6).

Although the present invention makes it possible to perform iontophoresis using an electrically conductive gel, it also has an advantage in that iontophoresis may be more conveniently performed using the cataplasma patch wet with water. This advantage is expected to obtain a considerable response in terms of ease of use when the cataplasma patch is used personally at home.

## Claims

1. A kit comprising:
a cataplasma patch, wherein a cataplasma composition is applied to a nonwoven fabric; said cataplasma composition comprising: a profen drug; a hydrophilic polymer; and a solubilizer, wherein the hydrophilic polymer is at least one selected from the group consisting of partially neutralized polyacrylate, and sodium carboxymethylcellulose; the profen drug is ibuprofen, loxoprofen, ketoprofen, dexibuprofen, flurbiprofen, or pranoprofen; the solubilizer is at least one of propylene glycol, polyethylene glycol, L-menthol, Pharmasolve and N-methyl-2-pyrrolidone; said cataplasma patch having a pH of 5 to 6;
an iontophoretic device configured to form an electric circuit in the cataplasma patch, to be applied to a local area of skin, and the skin so that an electric current flows;
electrically conductive gel to be applied or water to be sprayed onto the outer surface of the nonwoven fabric.

2. The kit according to claim 1;
the kit being configured such that the iontophoretic device is to be used with one hand by wetting the cataplasma composition with water, placing a negative electrode of the iontophoretic device in contact on the wet cataplasma composition, and bringing a positive electrode of the iontophoretic device into contact with the hand.

3. The kit according to claim 1;
comprising an electrically conductive gel composition comprising an ionic polymer;
wherein an ionic moiety of the ionic polymer in the electrically conductive gel composition is selected from among an anionic moiety or a cationic moiety,
the anionic moiety is at least one selected from the group consisting of carboxylate, nitrate, phosphate, and sulfate,
the cationic moiety is at least one selected from the group consisting of amine salt, imine salt, and ammonium salt,
the polymer is at least one selected from the group consisting of carboxylate, nitrate, phosphate, and sulfate, and
wherein the iontophoretic device is to be used with one hand by bringing a negative electrode of the iontophoretic device into contact with the electrically conductive gel composition applied onto the cataplasma composition and bringing a positive electrode of the iontophoretic device into contact with the hand.

4. The kit of any one claims 1 to 3, wherein said nonwoven fabric is a non-electrically conductive material.

## Patentansprüche

1. Kit umfassend:
ein Kataplasma-Pflaster, bei dem eine Kataplasma-Zusammensetzung auf einen Vliesstoff aufgebracht ist;
wobei die Kataplasma-Zusammensetzung umfasst: ein Profen-Arzneimittel; ein hydrophiles Polymer; und einen Lösungsvermittler, wobei das hydrophile Polymer mindestens eines ausgewählt aus der Gruppe bestehend aus teilweise neutralisiertem Polyacrylat und Natriumcarboxymethylcellulose ist; das Profen-Arzneimittel Ibuprofen, Loxoprofen, Ketoprofen, Dexibuprofen, Flurbiprofen oder Pranoprofen ist; der Lösungsvermittler mindestens eines aus Propylenglykol, Polyethylenglykol, L-Menthol, Pharmasolve und N-Methyl-2-Pyrrolidon ist; wobei das Kataplasma-Pflaster einen pH-Wert von 5 bis 6 aufweist;
eine iontophoretische Vorrichtung, die konfiguriert ist, einen elektrischen Stromkreis in dem Kataplasma-Pflaster, das auf einem lokalen Hautbereich aufzubringen ist, und der Haut, zu bilden, so dass ein elektrischer Strom fließt;
Elektrisch leitfähiges Gel, das aufzubringen ist, oder Wasser, das auf die Außenfläche des Vliesstoffs zu sprühen ist.

2. Kit nach Anspruch 1;
wobei das Kit so konfiguriert ist, dass die iontophoretische Vorrichtung mit einer Hand zu verwenden ist, indem die Kataplasma-Zusammensetzung mit Wasser angefeuchtet wird, eine negative Elektrode der iontophoretischen Vorrichtung in Kontakt mit der nassen Kataplasma-Zusammensetzung gebracht wird und eine positive Elektrode der iontophoretischen Vorrichtung in Kontakt mit der Hand gebracht wird.

3. Kit nach Anspruch 1;
umfassend eine elektrisch leitfähige Gelzusammensetzung umfassend ein ionisches Polymer;
wobei eine ionische Gruppe des ionischen Polymers in der elektrisch leitfähigen Gelzusammensetzung aus einer anionischen Gruppe oder einer kationischen Gruppe ausgewählt ist,
die anionische Gruppe mindestens eine ausgewählt aus der Gruppe bestehend aus Carboxylat, Nitrat, Phosphat und Sulfat ist,
die kationische Gruppe mindestens eine ausgewählt aus der Gruppe bestehend aus Aminsalz, Iminsalz und Ammoniumsalz ist,
das Polymer mindestens eines ausgewählt aus der Gruppe bestehend aus Carboxylat, Nitrat, Phosphat und Sulfat ist, und
wobei die iontophoretische Vorrichtung mit einer Hand zu verwenden ist, indem eine negative Elektrode der iontophoretischen Vorrichtung in Kontakt mit der elektrisch leitfähigen Gelzusammensetzung gebracht wird, die auf die Kataplasma-Zusammensetzung aufgebracht ist und eine positive Elektrode der iontophoretischen Vorrichtung in Kontakt mit der Hand gebracht wird.

4. Kit nach einem der Ansprüche 1 bis 3, wobei der Vliesstoff ein nicht elektrisch leitfähiges Material ist.

## Revendications

1. Un kit comprenant :
un timbre de cataplasme, dans lequel une composition de cataplasme est appliquée sur un tissu non tissé ;
ladite composition de cataplasme comprenant : un médicament profène ; un polymère hydrophile ; et un solubilisant, dans lequel le polymère hydrophile est au moins un choisi dans le groupe constitué de polyacrylate partiellement neutralisé et de carboxyméthylcellulose sodique ; le médicament profène est de l'ibuprofène, du loxoprofène, du kétoprofène, du dexibuprofène, du flurbiprofène ou du pranoprofène ; le solubilisant est au moins l'un du propylène glycol, du polyéthylène glycol, du L-menthol, du Pharmasolve et de la N-méthyl-2-pyrrolidone ; ledit timbre de cataplasme ayant un pH de 5 à 6 ;
un dispositif iontophorétique configuré pour former un circuit électrique dans le timbre de cataplasme, à appliquer sur une zone cutanée locale, et la peau de sorte qu'un courant électrique circule ;
un gel électroconducteur à appliquer ou de l'eau à pulvériser sur la surface externe du tissu non tissé.

2. Le kit selon la revendication 1 ;
le kit étant configuré de telle manière que le dispositif iontophorétique doit être utilisé avec une main en mouillant la composition de cataplasme avec de l'eau, en plaçant une électrode négative du dispositif iontophorétique en contact sur la composition de cataplasme mouillée, et en amenant une électrode positive du dispositif iontophorétique en contact avec la main.

3. Le kit selon la revendication 1 ;
comprenant une composition de gel électroconducteur comprenant un polymère ionique ;
dans lequel un groupement ionique du polymère ionique dans la composition de gel électroconducteur est choisi parmi un groupement anionique ou un groupement cationique,
le groupement anionique est au moins un choisi dans le groupe constitué de carboxylate, de nitrate, de phosphate et de sulfate,
le groupement cationique est au moins un choisi dans le groupe constitué de sel d'amine, de sel d'imine et de sel d'ammonium,
le polymère est au moins un choisi dans le groupe constitué de carboxylate, de nitrate, de phosphate et de sulfate, et
dans lequel le dispositif iontophorétique doit être utilisé avec une main en amenant une électrode négative du dispositif iontophorétique en contact avec la composition de gel électroconducteur appliquée sur la composition de cataplasme et en amenant une électrode positive du dispositif iontophorétique en contact avec la main.

4. Le kit selon l'une quelconque des revendications 1 à 3, dans lequel ledit tissu non tissé est un matériau non électroconducteur.
